# FASCICULE DE BREVET EUROPEEN

(11) **EP 0 931 059 B1**
(45) Date de publication et mention de la délivrance du brevet: **12.12.2001**
(21) Numéro de dépôt: 97940192.4
(22) Date de dépôt: 08.09.1997
(51) Int. Cl.: C07C 255/50

(54) **PROCEDE POUR LA PREPARATION D'UN CYANOBIPHENYLE**
VERFAHREN ZUR HERSTELLUNG EINES CYANOBIPHENYLS
METHOD FOR PREPARING A CYANOBIPHENYL

(30) Priorité: 09.09.1996 FR 9610970
(43) Date de publication de la demande: 28.07.1999
(73) Titulaire: SANOFI-SYNTHELABO, 75635 Paris Cédex 13 (FR)
(72) Inventeur: ALAMI, Mouad, F-77000 Bussy-Saint-Georges (FR); CAHIEZ, Gérard, F-75003 Paris (FR); CASTRO, Bertrand, F-34130 Saint Aunes (FR); RIGUET, Eric, F-91920 Les Ulis (FR)
(74) Mandataire: Le Guen, Gérard
(86) Numéro de dépôt international: FR9701577
(87) Numéro de publication internationale: WO9809941

(56) Documents cités:
- EP-A- 0 341 514
- EP-A- 0 470 794
- EP-A- 0 566 468
- EP-A- 0 571 770

## Description

La présente invention concerne un procédé de préparation d'un cyanobiphényle Plus particulièrement, l'invention a pour objet un procédé de préparation du o-(p-tolyl)benzonitrile de formule I 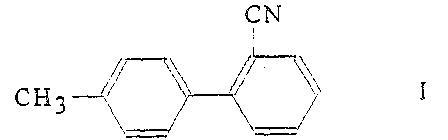 qui constitue l'intermédiaire clé dans la synthèse de nombreux principes actifs de médicaments agissant notamment contre l'hypertension par un mécanisme d'inhibition de l'angiotensine II.

Le o-(p-tolyl)benzonitrile, ci-après désigné plus brièvement orthotolylbenzonitrile ou OTBN, a été décrit pour la première fois dans EP 253310 et un certain nombre de procédés pour sa synthèse ont été récemment proposés.

Le procédé qui semble être le plus approprié pour la préparation de l'OTBN est décrit dans EP 566 468 et consiste en la réaction entre un o-halobenzonitrile avec un halogénure de p-tolylmagnésium en présence d'un sel manganeux, de préférence MnCl₂. Cette méthode, par rapport à celles précédemment connues a l'avantage de se dérouler en une seule étape avec des rendements d'environ 70% avant cristallisation. Elle donne, cependant comme sous-produit de réaction, le 4,4'-diméthylbiphényle résultant de la condensation de l'halogénure de p-tolylmagnésium sur lui-même.

EP 341 514 décrit la synthèse d'un composé biarylique par couplage de deux composés aromatiques halogénés par action d'un métal (zinc, manganèse ou magnésium) en présence i) d'un catalyseur qui est un composé du nickel, ii) d'un promoteur qui peut être un halogénure de manganèse, un halogénure de magnésium, un halogénure de zinc, un halogénure de métal alcalino-terreux ou un halogénure de métal alcalin et iii) d'un ligand phosphoré.

Ce procédé ne met nullement en jeu un réactif de type Grignard et nécessite la présence d'un ligand phosphoré.

Il a été maintenant trouvé que si la réaction entre l'halogénure de p-tolyl magnésium et le o-halobenzonitrile est effectuée en présence d'un sel manganeux et de traces d'un sel de palladium (II), l'OTBN est obtenu avec un rendement d'au moins environ 92% alors que l'impureté 4,4'-diméthylbiphényle chute au dessous d'environ 2,5 %.

Ainsi, la présente invention a pour objet un procédé pour la préparation de l'o-(p-tolyl)benzonitrile, caractérisé en ce qu'on traite un o-halobenzonitrile, de préférence l'o-bromobenzonitrile avec un halogénure de p-tolylmagnésium en présence d'un sel manganeux et d'un co-catalyseur contenant un métal de transition, de préférence un sel de palladium (II).

La réaction de couplage selon l'invention est effectuée dans un solvant du type éther tel que le méthyl-t-butyl éther, le dibutyl éther, le dioxane ou le tétrahydrofurane, la température de réaction pouvant varier de -10 à 60°C, selon le solvant employé.

Cette réaction conduit transitoirement à la formation d'un complexe que l'on hydrolyse selon les procédures connues, par exemple au moyen d'un acide tel que l'acide chlorhydrique.

Quant au sel manganeux, il s'agit de préférence de MnCl₂ ou de MnCl₄Li₂, ce dernier pouvant être formé *in situ* par addition de deux équivalents molaires de LiCl et d'un équivalent molaire de MnCl₂.

Ce sel manganeux intervient dans la réaction à raison de 0,5 à 1,3 équivalent molaire par équivalent molaire de o-halobenzonitrile de départ.

Le métal de transition, formant le co-catalyseur, est avantageusement le nickel, le cobalt, le platine ou notamment le palladium.

Comme co-catalyseur contenant un métal de transition on utilise préférentiellement un sel de palladium (II), notamment le nitrate, le chlorure, l'acétate, le bromure, le sulfate ou analogues, le chlorure (PdCl₂) et l'acétate (CH₃-COO-Pd-OOC-CH₃) étant particulièrement avantageux. De préférence, le sel de palladium est complexé par exemple avec au moins un composé organophosphoré comprenant du phosphore trivalent. Plus particulièrement, on peut mentionner les complexes du palladium, tels que les bis(triphénylphosphine)dichloro-, bis(tributylphosphine)dichloro-, bis(tricyclohexylphosphine)dichloro-, diallyltriphénylphosphinedichloro-, triphénylphosphinepipéridinedichloro-, bis(cyclohexyloxime)dicarbonyl-, 1,5,9-cyclododécatriènedichloro-, bis(triphénylphosphine)dicarbonyl-, bis(triphénylphosphine)diacétate-, bis(triphénylphosphine)-sulfate-, 2,4-pentanedione, tétrakis(triphénylphosphine)-palladium. Parmi ceux-ci, les complexes de palladium (II) sont particulièrement avantageux, le 1,3-bis(diphénylphosphino)propane (dppp) complexé avec le chlorure de palladium (II) ou l'acétate de palladium (II) étant préféré.

Les sels de palladium et les composés organosphosphorés peuvent être ajoutés séparément au milieu réactionnel.

Dans ce cas, la quantité de composé organophosphoré est, de préférence, suffisante pour former in situ le co-catalyseur. sous forme de complexe avec le palladium présent. Ledit complexe est en général préparé de façon à ce que le rapport P/Pd soit environ 1/1, mais un tel rapport peut varier entre 0,5/1 et 2/1 sans altérer significativement le résultat du procédé.

Ce co-catalyseur est présent en très faibles quantités dans le mélange réactionnel, à savoir de 0,001 à 2% molaire par mole de o-halobenzonitrile de départ.

Selon un mode opératoire préférentiel, l'halogénure de p-tolylmagnésium est en quantités équimolaires ou en faible excès (1 à 1,5 mole) par rapport à l'o-halobenzonitrile. En outre, le catalyseur manganeux préféré (MnCl₄Li₂) est formé. soit *in situ,* en quantités équimolaires ou en faible excès (1 à 1,5 mole) par rapport à l'o-halobenzonitrile, soit extemporanément avant addition au milieu réactionnel.

MnCl₄Li₂ est préparé en faisant réagir un équivalent de MnCl₂ avec deux équivalents de LiCl.

La réaction peut être conduite dans le tétrahydrofurane en ajoutant, à la température de 10°C, le co-catalyseur et l'o-halobenzonitrile éventuellement en solution dans le tétrahydrofurane à une solution de tétrahydrofurane contenant l'halogénure de p-tolylmagnésium et le catalyseur manganeux. Cette réaction qui est exothermique, peut être contrôlée en réglant la vitesse d'addition du dérivé de benzonitrile et du co-catalyseur de façon à la maintenir inférieure à 35°C.

D'une manière alternative, la réaction peut être également conduite en ajoutant l'halogénure de p-tolylmagnésium dans, par exemple, le tétrahydrofurane à un mélange d'o-halobenzonitrile, de co-catalyseur et de catalyseur manganeux dans, par exemple, le tétrahydrofurane. Dans ce cas, la température de réaction peut être mieux contrôlée et l'addition d'haiogénure de p-tolylmagnésium peut être effectuée même à une température plus élevée, de l'ordre de 50-55°C, de façon à diminuer la durée de la réaction et la quantité de co-catalyseur employée.

Pour améliorer le déroulement de la réaction, il peut être avantageux cependant d'ajouter un co-solvant au mélange contenant le catalyseur manganeux. Ce co-solvant est de préférence un autre éther ou di-éther, par exemple le diméthoxyéthane.

Selon le mode opératoire préférentiel ci-dessus, l'hydrolyse est effectuée *in situ* par de l'acide chlorhydrique et l'OTBN ainsi formé est isolé selon les techniques conventionnelles, par exemple par extraction avec un solvant convenable, évaporation du solvant et purification par cristallisation dans de l'éthanol ou par chromatographie.

L'OTBN est ainsi obtenu avec des rendements très élevés, de 92 à 98% selon les proportions des réactifs employés. Il contient des quantités très faibles de 4,4'-diméthylbiphényle, généralement inférieures à 2,5 %.

La quantité de 4,4'-diméthylbiphényle qui se forme selon le procédé de la présente invention a été comparée à celle qui se forme selon le procédé décrit dans EP 566 468. Ainsi en opérant :
- selon EP 566 468, à savoir en utilisant le seul MnCl₂ en tant que catalyseur, dans une série d'essais dans les mêmes conditions, on a obtenu du sous-produit 4,4'-diméthylbiphényle avec un rendement de 8 à 12% par rapport au bromure de p-tolylmagnésium, soit 6,5 à 10% en poids de 2-(p-tolyl)benzonitrile produit final;
- selon la présente invention, à savoir en utilisant MnCl₂ et PdCl₂/dppp en tant que catalyseur et co-catalyseur, dans une série d'essais dans les mêmes conditions, on a obtenu du sous-produit 4,4'-diméthylbiphényle avec un rendement de 0,5 à 1% par rapport au bromure de p-tolylmagnésium, soit au maximum 0,65 % en poids de produit final.

Le co-catalyseur contenant un métal de transition peut être également un sel de colbalt, de nickel, ou de platine comme indiqué précédemment.
Dans le cas d'un co-catalyseur contenant du nickel, on utilise généralement un sel de nickel (II) tel que le chlorure, ou l'acétylacétonate de nickel. Ce sel est préférentiellement complexé avec au moins un composé organophosphoré comprenant du phosphore trivalent tel qu'une phosphine, par exemple la triphénylphosphine. Le sel de nickel et le composé organosphosphoré peuvent être ajoutés séparément au milieu réactionnel.
Ce co-catalyseur contenant du nickel est avantageusement prétraité avec un agent réducteur tel qu'un hydrure par exemple l'hydrure de dibutylaluminium ou l'hydrure de diisobutylaluminium ou encore avec un halogénure de méthylmagnésium par exemple le chlorure de méthylmagnésium, de manière à former des catalyseurs contenant du Ni(O) tel que Ni [P(C₆H₅)₃]₄.
Des systèmes comprenant l'acétylacétonate de nickel, la triphénylphosphine et l'hydrure de diisobutylaluminium se sont révélés particulièrement intéressants. Les exemples non limitatifs suivants illustrent l'invention. Dans ces exemples, les pourcentages molaires en co-catalyseur sont calculés par rapport à la quantité d' ortho-halobenzonitrile.

### EXEMPLE 1

Sous atmosphère d'azote et à la température ambiante, on ajoute successivement, à 2 ml de tétrahydrofurane anhydre, du MnCl₂ (0,65 g, 5,14 mmol) et du LiCl (0,44 g, 10,28 mmol). On agite le mélange jusqu'à dissolution des sels (formation de MnCl₄Li₂). On ajoute alors une solution de chlorure de p-tolylmagnésium dans le tétrahydrofurane (1,80N, 2,86 ml, 5,14 mmol) de manière telle que la température soit maintenue entre -10°C et 0°C. On additione ensuite rapidement à 0°C du diméthoxyéthane (1 ml, 10,28 mmol) et on maintient sous agitation pendant 5 mn à +10°C le composé organomanganeux ainsi obtenu.
On ajoute alors du PdCl₂/dppp : 1/1 (0,023 g, 1 mol %) puis de l'o-bromobenzonitrile (0,72 g, 3,955 mmol). La température s'élève de +10 à +30°C en 15 mn, puis redescend lentement à +25°C.
Après 3 heures d'agitation à la température ambiante, on hydrolyse le mélange réactionnel à l'aide d'une solution d'acide chlorhydrique IN (15 ml). Après extraction à l'éther éthylique, on sèche la phase organique sur carbonate de potassium, filtre puis évapore sous vide. On purifie ensuite par chromatographie (silice: 20 g; éluant éther de pétrole/acétate d'éthyle = 95/5) l'huile ainsi formée. On récolte alors, avec un rendement de 96%, le o-(p-tolyl)benzonitrile ainsi obtenu, sous forme de cristaux blanchâtres.
Point de fusion: +48°C
RMN ¹H (CDCl₃) δ 2,42 (s, 3H, CH₃)
RMN¹³C (CDCl₃) δ 21,03; 110,85; 118,70; 127,09; 128,39; 129,22; 129,74; 132,60; 133,47; 135,03; 138,43; 145,20.

### EXEMPLES 2 et 3

En opérant comme décrit dans l'Exemple 1, en utilisant 0,5 équivalents de MnCl₄Li₂ et 1,3 équivalent de chlorure de p-tolylmagnésium pour un équivalent de o-halobenzonitrile et en faisant varier la quantité de PdCl₂/dppp : 1/1, on a obtenu les rendements en o-(p-tolyl)benzonitrile indiqués dans le Tableau 1.

**TABLEAU 1**

| Exemple | PdCl₂/dppp:1/1 (% molaire) | Temps d'agitation à la t° ambiante | Rendement en produit isolé |
|---|---|---|---|
| 2 | 1 | 90 minutes | 95% |
| 3 | 0,5 | 5 heures | 92% |

### EXEMPLE 4

On agite à la température ambiante, une suspension de MnCl₂ (0,25 g, 1,98 mmol) et de LiCl (0,17 g, 3,955 mmol) dans un mélange de 2 ml de tétrahydrofurane anhydre et 0,38 ml de diméthoxyéthane (3,955 mmol).
Après environ 30 minutes d'agitation, on obtient un milieu homogène. On ajoute alors le PdCl₂/dppp : 1/1 (0,023 g, 1 mol %) et l'o-bromobenzonitrile (0,72 g, 3,955 mmol), puis l'on additionne en 2 heures, à la température ambiante, une solution de chlorure de p-tolylmagnésium dans le tétrahydrofurane (1,80N, 2,86 ml, 5,14 mmol). Après 90 minutes d'agitation, on traite le milieu réactionnel comme décrit dans l'Exemple 1. On obtient ainsi le o-(p-tolyl)benzonitrile avec un rendement en produit isolé de 95%.

### EXEMPLE 5

On opère exactement comme décrit dans l'Exemple 4, en remplaçant le complexe PdCl₂/dppp par un mélange 1:1 d'acétate de palladium (II) et de dppp. On obtient ainsi le o-(p-tolyl)benzonitrile avec un rendement en produit isolé de 94%.

### EXEMPLE 6

On opère exactement comme décrit dans l'Exemple 4, mais on remplace le complexe PdCl₂/dppp par un mélange 1:1 PdCl₂·2LiCl et de dppp. On obtient ainsi le o-(p-tolyl)benzonitrile avec un rendement en produit isolé de 95%.

### EXEMPLE 7

On agite, à la température ambiante, une suspension de MnCl₂ (0,25 g, 1,98 mmol) et de LiCl (0,17 g, 3,955 mmol) dans un mélange de 2 ml de tétrahydrofurane anhydre et 0,38 ml de diméthoxyéthane (3,955 mmol) jusqu'à obtention, après environ 30 minutes, d'un milieu homogène. On ajoute alors le complexe PdCl₂/dppp (0,0023 g, 0,1 mol %) et l'o-bromobenzonitrile (0,72 g, 3,955 mmol), puis on additionne en 30 minutes une solution de chlorure de p-tolylmagnésium dans le tétrahydrofurane (1,80N, 2,86 ml, 5,14 mmol). Après 30 minutes d'agitation, on traite le milieu réactionnel comme décrit dans l'Exemple 4. On obtient ainsi le o-(p-tolyl)benzonitrile avec un rendement en produit isolé de 95%.

## Revendications

1. Procédé pour la préparation de l'o-(p-tolyl)benzonitrile **caractérisé en ce qu'**on traite un o-halobenzonitrile avec un halogénure de p-tolylmagnésium en présence d'un sel manganeux et d'un co-catalyseur contenant un métal de transition.

2. Procédé selon la revendication 1, **caractérisé en ce que** l'o-halobenzonitrile est l'o-bromobenzonitrile.

3. Procédé selon la revendication 1 ou 2, **caractérisé en ce que** le sel manganeux est MnCl₂ ou MnCl₄Li₂.

4. Procédé selon une des revendications 1 à 3 **caractérisé en ce que** le co-catalyseur contenant un métal de transition est un sel de palladium, un sel de cobalt, un sel de nickel ou un sel de platine.

5. Procédé selon une des revendications 1 à 3 **caractérisé en ce que** le co-catalyseur contenant un métal de transition est un sel de palladium (II).

6. Procédé selon la revendication 5, **caractérisé en ce que** le sel de palladium (II) est le chlorure de palladium (II) ou l'acétate de palladium (II).

7. Procédé selon l'une des revendications 4 à 6, **caractérisé en ce que** le sel de palladium est ajouté au milieu réactionnel avec un composé organophosphoré comprenant du phosphore trivalent.

8. Procédé selon l'une des revendications 4 à 7, **caractérisé en ce que** le sel de palladium est sous forme de complexe avec un composé organosphosporé comprenant du phosphore trivalent.

9. Procédé selon la revendication 8, **caractérisé en ce que** le sel de palladium est sous forme de complexe entre le 1,3 - bis (diphénylphosphino) propane et le chlorure ou l'acétate de palladium (II).

10. Procédé selon la revendication 4, **caractérisé en ce que** le sel de nickel est l'acétylacétonate de nickel.

11. Procédé selon la revendication 4 ou 10, **caractérisé en ce que** le sel de nickel est ajouté au milieu réactionnel avec un composé organophosphoré comprenant du phospore trivalent.

12. Procédé selon une des revendications 4, 10 ou 11 **caractérisé en ce que** le sel de nickel est sous forme de complexe avec un composé organophosphoré comprenant du phosphore trivalent.

13. Procédé selon une des revendications 4,10,11 ou 12 **caractérisé en ce que** le sel de nickel est prétraité avec un agent réducteur.

14. Procédé selon une des revendications 1 à 13, **caractérisé en ce que** la réaction est conduite dans un solvant du type éther.

15. Procédé selon la revendication 14, **caractérisé en ce que** le solvant est le tétrahydrofurane.

16. Procédé selon une des revendications 1 à 15, **caractérisé en ce que** la réaction est conduite dans un solvant du type éther et un co-solvant du type diéther.

17. Procédé selon la revendication 16, **caractérisé en ce que** le solvant est le tétrahydrofurane et le co-solvant est le diméthoxyéthane.

## Patentansprüche

1. Verfahren zur Herstellung von o-(p-Tolyl)-benzonitril, **dadurch gekennzeichnet, daß** man ein o-Halogenbenzonitril mit einem p-Tolylmagnesiumhalogenid in Gegenwart eines Mangan(II)-salzes und eines Co-Katalysators, der ein Übergangsmetall enthält, behandelt.

2. Verfahren nach Anspruch 1, **dadurch gekennzeichnet, daß** das o-Halogenbenzonitril o-Brombenzonitril ist.

3. Verfahren nach Anspruch 1 oder 2, **dadurch gekennzeichnet, daß** das Mangan(II)-salz MnCl₂ oder MnCl₄Li₂ ist.

4. Verfahren nach einem der Ansprüche 1 bis 3, **dadurch gekennzeichnet, daß** der ein Übergangsmetall enthaltende Co-Katalysator ein Palladiumsalz, ein Kobaltsalz, ein Nickelsalz oder ein Platinsalz ist.

5. Verfahren nach einem der Ansprüche 1 bis 3, **dadurch gekennzeichnet, daß** der ein Übergangsmetall enthaltende Co-Katalysator ein Palladium(II)-salz ist.

6. Verfahren nach Anspruch 5, **dadurch gekennzeichnet, daß** das Palladium(II)-salz Palladium(II)-chlorid oder Palladium(II)-acetat ist.

7. Verfahren nach einem der Ansprüche 4 bis 6, **dadurch gekennzeichnet, daß** das Palladiumsalz zusammen mit einer dreiwertigen Phosphor enthaltenden phosphororganischen Verbindung zu dem Reaktionsmedium zugesetzt wird.

8. Verfahren nach einem der Ansprüche 4 bis 7, **dadurch gekennzeichnet, daß** das Palladiumsalz in Form eines Komplexes mit einer dreiwertigen Phosphor enthaltenden phosphororganischen Verbindung vorliegt.

9. Verfahren nach Anspruch 8, **dadurch gekennzeichnet, daß** das Palladiumsalz in Form eines Komplexes zwischen 1,3-Bis(diphenylphosphino)-propan und Palladium(II)-chlorid oder -acetat vorliegt.

10. Verfahren nach Anspruch 4, **dadurch gekennzeichnet, daß** das Nickelsalz Nickelacetylacetonat ist.

11. Verfahren nach Anspruch 4 oder 10, **dadurch gekennzeichnet, daß** das Nikkelsalz zusammen mit einer dreiwertigen Phosphor enthaltenden phosphororganischen Verbindung zu dem Reaktionsmedium zugesetzt wird.

12. Verfahren nach einem der Ansprüche 4, 10 oder 11, **dadurch gekennzeichnet, daß** das Nickelsalz in Form eines Komplexes mit einer dreiwertigen Phosphor enthaltenden phosphororganischen Verbindung vorliegt.

13. Verfahren nach einem der Ansprüche 4, 10, 11 oder 12, **dadurch gekennzeichnet, daß** das Nickelsalz mit einem Reduktionsmittel vorbehandelt wird.

14. Verfahren nach einem der Ansprüche 1 bis 13, **dadurch gekennzeichnet, daß** die Reaktion in einem Lösungsmittel vom Typ eines Ethers durchgeführt wird.

15. Verfahren nach Anspruch 14, **dadurch gekennzeichnet, daß** das Lösungsmittel Tetrahydrofuran ist.

16. Verfahren nach einem der Ansprüche 1 bis 15, **dadurch gekennzeichnet, daß** die Reaktion in einem Lösungsmittel vom Typ eines Ethers und einem Co-Lösungsmittel vom Typ eines Diethers durchgeführt wird.

17. Verfahren nach Anspruch 16, **dadurch gekennzeichnet, daß** das Lösungsmittel Tetrahydrofuran und das Co-Lösungsmittel Dimethoxyethan sind.

## Claims

1. Process for preparing o-(p-tolyl)benzonitrile, **characterised in that** an o-halobenzonitrile is treated with a p-tolylmagnesium halide in the presence of a manganese salt and a co-catalyst containing a transition metal.

2. Process according to claim 1, **characterised in that** the o-halobenzonitrile is o-bromobenzonitrile.

3. Process according to claim 1 or 2, **characterised in that** the manganese salt is MnCl₂ or MnCl₄Li₂.

4. Process according to one of claims 1 to 3, **characterised in that** the co-catalyst containing a transition metal is a palladium salt, a cobalt salt, a nickel salt or a platinum salt.

5. Process according to one of claims 1 to 3; **characterised in that** the co-catalyst containing a transition metal is a palladium (II) salt.

6. Process according to claim 5, **characterised in that** the palladium (II) salt is palladium (II) chloride or palladium (II) acetate.

7. Process according to one of claims 4 to 6, **characterised in that** the palladium salt is added to the reaction medium with an organophosphorus compound containing trivalent phosphorus.

8. Process according to one of claims 4 to 7, **characterised in that** the palladium salt is in the form of a complex with an organophosphorus compound containing trivalent phosphorus.

9. Process according to claim 8, **characterised in that** the palladium salt is in the form of a complex between 1,3-bis(diphenylphosphino)propane and palladium (II) chloride or acetate.

10. Process according to claim 4, **characterised in that** the nickel salt is nickel acetyl acetonate.

11. Process according to claim 4 or 10, **characterised in that** the nickel salt is added to the reaction medium with an organophosphorus compound containing trivalent phosphorus.

12. Process according to one of claims 4, 10 or 11, **characterised in that** the nickel salt is in the form of a complex with an organophosphorus compound containing trivalent phosphorus.

13. Process according to one of claims 4, 10, 11 or 12, **characterised in that** the nickel salt is pretreated with a reducing agent.

14. Process according to one of claims 1 to 13, **characterised in that** the reaction is carried out in an ether-type solvent.

15. Process according to claim 14, **characterised in that** the solvent is tetrahydrofuran.

16. Process according to one of claims 1 to 15, **characterised in that** the reaction is carried out in an ether-type solvent and a diether-type co-solvent.

17. Process according to claim 16, **characterised in that** the solvent is tetrahydrofuran and the co-solvent is dimethoxyethane.
